# FASCICULE DE BREVET EUROPEEN

(11) **EP 0 866 875 B1**
(45) Date de publication et mention de la délivrance du brevet: **16.10.2002**
(21) Numéro de dépôt: 96942412.6
(22) Date de dépôt: 13.12.1996
(51) Int. Cl.: C12P 19/04, C12N 1/06, A61K 7/48

(54) **PROCEDE DE PRODUCTION DE GLYCOGENE OU D'UN EXTRAIT RICHE EN GLYCOGENE A PARTIR DE CELLULES DE LEVURE ET COMPOSITION COSMETIQUE LES CONTENANT**
VERFARHEN ZUR HERSTELLUNG VON GLYCOGEN ODER GLYCOGENREICHEM HEFEEXTRAKT AUS HEFEZELLEN UND KOSMETISCHE ZUSAMMENSETZUNGEN, DIE DIESE ENTHALTEN
METHOD FOR PRODUCING GLYCOGEN OR A GLYCOGEN-ENRICHED EXTRACT FROM YEAST CELLS, AND COSMETIC COMPOSITION CONTAINING SAME

(30) Priorité: 14.12.1995 FR 9515310
(43) Date de publication de la demande: 30.09.1998
(73) Titulaire: Cognis France S.A., 31360 Saint Martory (FR)
(72) Inventeur: PAULY, Gilles, F-54280 Seichamps (FR); PAULY, Marc, F-57170 Château-Salins (FR); ENGASSER, Jean-Marc, F-54710 Ludres (FR); GHOUL, Mohamed, F-54000 Nancy (FR)
(74) Mandataire: Nuss, Pierre
(86) Numéro de dépôt international: FR9602008
(87) Numéro de publication internationale: WO97021828

(56) Documents cités:
- DD-A- 266 584
- FR-A- 2 447 193
- FR-A- 2 486 400
- DATABASE WPI Section Ch, Week 9311 Derwent Publications Ltd., London, GB; Class D16, AN 93-087944 XP002012463 & JP 05 030 961 A (TOTO LTD) , 9 Février 1993
- DATABASE WPI Section Ch, Week 8737 Derwent Publications Ltd., London, GB; Class D21, AN 87-259754 XP002012566 & JP 62 178 505 A (MARUZEN KASEI KK) , 5 Août 1987

## Description

La présente invention concerne le domaine de la cosmétologie, notamment la production de matières premières pour la cosmétique, et a pour objet un procédé de production de glycogène ou d'extraits riches en glycogène à partir de cellules de levure, ainsi que l'utilisation de ce glycogène ou de ces extraits dans des compositions cosmétiques et les compositions résultantes.

Il est connu que le glycogène, polysaccharide composé de glucose et réserve énergétique des cellules vivantes, est particulièrement abondant dans les cellules de levures.

On connaît déjà différents procédés de désintégration et de traitement de cellules de levure pour la production de protéines, notamment recombinantes, comprenant la désintégration desdites cellules, la séparation des débris cellulaires, la récupération des protéines et la purification de ces dernières.

Néanmoins, ces procédés connus ne permettent pas une extraction préférentielle de glycogène avec un rendement élevé.

On connaît également des procédés d'hydrolyses de cellules de levures par traitement thermique, enzymatique ou chimique permettant de produire des extraits de levures à des fins alimentaires ou thérapeutiques.

Toutefois, ces procédés connus, souvent basés sur des hydrolyses acides, conduisent à des préparations sous forme d'hydrolysats contenant l'ensemble des protéines et des polysaccharides des levures, et ne peuvent pas fournir des extraits de levures spécifiquement enrichis en glycogène.

Il est connu, en outre, d'extraire du glycogène de bactéries marines, en soumettant ces dernières à un traitement thermique et chimique, à une précipitation à l'acide trichloracétique et à des précipitations consécutives à l'éthanol.

Ces extractions, effectuées à l'échelle du laboratoire, ont uniquement pour but de permettre l'analyse de la structure et de la composition du glycogène soluble et ne sont applicables qu'au type spécifique de cellules visé, sans être applicables à des cellules présentant des parois plus résistantes.

Il existe également déjà des procédés de perméabilisation des parois de cellules de levure et d'hydrolyse du glycogène intracellulaire en glucose, afin d'en mesurer le taux de présence, ces procédés ne pouvant toutefois pas assurer une extraction du glycogène en tant que tel, notamment sous forme non hydrolysée.

Par ailleurs, le document FR-A-2 486 400 décrit notamment un procédé de production de glucanes extraits de levure et dépourvus de glycogène et leur utilisation dans des crèmes dermiques et le document WPI/Derwent. AN : 87-259754 (37) mentionne l'utilisation de glycogène extrait de tissus animaliers dans une composition cosmétique.

Le problème posé à la présente invention consiste, par conséquent, à concevoir un procédé de production de glycogène ou d'extraits riches en glycogène à partir de cellules de levure, pouvant être aisément mis en oeuvre industriellement en vue de la production de glycogène en grande quantité, présentant un rendement important par rapport à la quantité de matière première traitée, permettant d'extraire le glycogène soluble et le glycogène insoluble (lié aux protéines de la paroi des cellules) et donc d'obtenir des extraits à teneur variable en glycogène, notamment de valeur très élevée et sous forme non hydrolysée, et pouvant, le cas échéant, utiliser des levures résiduelles de procédés de fermentation.

Ce problème est précisément résolu par le procédé de production selon l'invention, caractérisé en ce qu'il consiste essentiellement à prendre une quantité donnée de cellules de levure, fournie par une culture spécifique ou récupérée en tant que résidus d'un procédé de fermentation, à soumettre lesdites cellules de levure à une opération d'enrichissement en glycogène intracellulaire en présence d'une source de carbone, à bloquer le métabolisme desdites cellules de levure, à désintégrer au moins partiellement les membranes desdites cellules de levure et à soumettre les substances intracellulaires libérées à une ou des précipitation(s) globale(s) ou sélective(s), le cas échéant après application d'une opération de fractionnement, de concentration, de déminéralisation et/ou de décoloration.

Les cellules de levure précitées peuvent consister, par exemple, en des levures récupérées à différents stades de procédés existants de production de levure ou d'utilisation de levure, par exemple au cours de la production de la bière.

Toutefois, lesdites cellules de levure peuvent également être obtenues par des procédés de propagation spécifiques effectués dans des fermenteurs contenant un milieu nutritif permettant la croissance des levures. La propagation des levures peut être effectuée en mode discontinu, en ajoutant tous les éléments du milieu nutritif au début de la culture, ou en mode semi-continu, en apportant les éléments nutritionnels au cours de la culture et tout au long de celle-ci, de façon continue ou intermittente.

Les levures utilisées, telles que les levures de brasserie ou de boulangerie, présentent préférentiellement une teneur en glycogène, avant enrichissement, comprise entre 1 % à 40 % (rapport pondéral glycogène/poids sec de cellules), cette teneur initiale en glycogène déterminant notamment la durée de la phase d'enrichissement.

Conformément à une première caractéristique de l'invention, ladite opération d'enrichissement en glycogène peut comprendre une première phase de revivification durant laquelle les cellules de levure sont disposées dans un milieu nutritif comprenant notamment une source de carbone, d'azote et de phosphore, des éléments minéraux et des vitamines, et une seconde phase de maturation durant laquelle les cellules de levure sont disposées dans un milieu nutritif contenant au moins une source de carbone, préférentiellement un sucre tel que le glucose ou le saccharose, à une concentration comprise entre 1 et 700 g par litre de milieu nutritif, une source d'azote, préférentiellement de l'ammoniaque et/ou divers éléments minéraux.

Durant cette opération d'enrichissement, les cellules de levure placées sur ou dans un milieu nutritif transforment la source de carbone présente dans ledit milieu en glycogène intracellulaire, ce dans des conditions aérobies ou anaérobies.

Les phases de revivification et de maturation peuvent être effectuées en mode discontinu, en ajoutant tous les éléments du milieu nutritif adapté à la phase considérée au début de chacune desdites phases de la culture, ou en mode semi-continu, en apportant les éléments nutritionnels au cours desdites phases de la culture et tout au long de celles-ci, de manière continue ou intermittente.

La phase de maturation est préférentiellement conduite dans des conditions de limitation en source d'azote, de chocs thermiques ou d'excès de sels minéraux. Sa durée peut varier de quelques minutes à une vingtaine d'heures environ en fonction de la teneur de glycogène intracellulaire souhaitée (généralement de 10 à 40 % en pourcentage pondéral par rapport à la masse des cellules de levure séchées).

Selon une autre caractéristique de l'invention, le blocage du métabolisme des cellules de levure, en vue de la désactivation des processus enzymatiques de dégradation et de consommation endogène de glycogène, est obtenu en effectuant un traitement thermique à une température comprise entre 60° C et 180° C pendant une durée comprise entre 30 secondes et 200 minutes ou un traitement chimique au moyen du Na₂CO₃ ou au moyen d'un solvant organique, tel que l'éthanol ou le propanol par exemple.

Il peut également être prévu, conformément à l'invention, d'effectuer, simultanément ou successivement, un traitement thermique et un traitement chimique des cellules de levure en vue d'obtenir la désactivation précitée.

Le blocage du métabolisme des cellules de levure est préférentiellement effectué lorsque ces dernières ont atteint leur teneur maximale en glycogène au cours de l'opération d'enrichissement, l'occurrence et la valeur de cette teneur maximale dépendant notamment de la nature et de l'âge desdites cellules.

Selon un premier mode de réalisation de l'invention, la désintégration des parois des cellules de levure peut être réalisée par broyage mécanique par l'intermédiaire d'un homogénéisateur haute pression, d'un broyeur à billes ou d'une French presse.

Selon un deuxième mode de réalisation de l'invention, la désintégration des parois des cellules de levure peut être réalisée au moyen de traitements non mécaniques tels que des traitements thermiques constitués de cycles répétitifs de congélation/décongélation, des traitements enzymatiques mettant en oeuvre des préparations à base de protéases et d'hydrolases et des traitements chimiques au moyen de solvants organiques, ces traitements pouvant être mis en oeuvre isolément ou de manière combinée.

Selon un troisième mode de réalisation de l'invention, la désintégration des parois des cellules de levure peut être réalisée en combinant le broyage mécanique et au moins un type de traitement non mécanique.

L'opération de désintégration des cellules de levure, qui permet de perméabiliser ou d'éclater la paroi des cellules et de libérer les substances intracellulaires, peut être effectuée avec une intensité variable en fonction de la nature de la levure et de la qualité souhaitée pour l'extrait de glycogène.

En effet, une augmentation du broyage peut permettre d'extraire davantage de glycogène, notamment celui lié aux protéines de la paroi cellulaire, et d'aboutir à une plus grande solubilisation des autres constituants des parois cellulaires.

Afin d'obtenir un extrait final de bonne qualité, les substances intracellulaires libérées après désintégration des parois des cellules de levure peuvent être soumises à au moins un traitement de fractionnement, de concentration et/ou de purification tel que la microfiltration, l'ultrafiltration ou la nanofiltration, l'électrodialyse, l'adsorption par échange d'ion ou l'adsorption par du charbon actif et la cryoconcentration.

Les traitements précités permettent respectivement de fractionner les constituants issus du broyage des cellules de levure, notamment les macromolécules glucidiques, protéiques et nucléiques, de concentrer les polysaccharides et les protéines, de réduire la teneur en petites molécules (sucres, acides aminés et peptides) et en éléments minéraux solubles et de décolorer la solution résultant du broyage.

Conformément à une autre caractéristique de l'invention, les substances intracellulaires libérées sont avantageusement soumises à une précipitation par des acides, tels que l'acide chlorhydrique ou l'acide trichloroacétique, effectuée à un pH compris entre 1 et 6 et à une température comprise entre 0° C et 60° C.

En variante, les substances intracellulaires libérées peuvent être soumises à une précipitation par des sels, tels que le sulfate d'ammonium, effectuée à un pH compris entre 1 et 9, à une température comprise entre 0° C et 30° C et avec une teneur en sels comprise entre 10 % et 80 %.

Selon une autre variante encore de réalisation de l'invention, les substances intracellulaires libérées peuvent également être soumises à une précipitation par des alcools, préférentiellement l'éthanol ou l'isopropanol, effectuée à un pH compris entre 1 et 9, à une température comprise entre 0° C et 30° C et avec une teneur en alcool, en rapport volumique, comprise entre 10 % et 80%.

Lorsque les cellules de levure traitées par le présent procédé sont récupérées en tant que résidus d'un procédé de fermentation, il peut être avantageusement prévu de soumettre les cellules de levure, avant l'opération d'enrichissement en glycogène, à au moins un lavage à l'eau à une température comprise entre 10° C et 60° C, éventuellement avec addition d'un agent chimique tel que Na₂CO₃, puis d'extraire et de concentrer lesdites cellules de levure, par centrifugation, décantation, filtration et/ou microfiltration.

Cette ou ces opérations de lavages permettent d'éliminer les substances absorbées par les cellules de levure ou présentes dans la suspension contenant ces dernières.

Ainsi, dans le cas de cellules de levure issues de procédés de brasserie, ce lavage permet d'éliminer les extraits de houblon.

Conformément à une autre caractéristique de l'invention, les substances intracellulaires libérées peuvent être soumises à au moins un traitement d'hydrolyse enzymatique sélective effectué au moyen de préparations adaptées contenant des protéases, des glucanases, des mannases ou un mélange de deux ou de plusieurs des enzymes précitées.

Ccs traitements d'hydrolyse permettent de réduire, voire d'annuler, la teneur de certaines macromolécules glucidiques et/ou protéiques présentent dans les extraits de levure.

Enfin, le procédé conformément à l'invention peut également consister à concentrer, à stabiliser et à conditionner les compositions obtenues par précipitation des substances intracellulaires libérées après désintégration des cellules de levure.

A cet effet, il peut être procédé à des opérations d'évaporation ou de filtration membranaire, à des ajouts d'adjuvants et/ou à des opérations de séchage et d'atomisation ou de lyophilisation.

Selon la nature des levures utilisées et des opérations de traitement précitées mises en oeuvre, le procédé de production et d'extraction selon l'invention peut conduire à des compositions de glycogène contenant des proportions variables de glycogène et de substances annexes.

Ainsi, les extraits obtenus au moyen du procédé selon l'invention peuvent avoir une teneur pondérale en glycogène, à l'état sec, comprise entre 5 % et 99,9 %, le rendement d'extraction étant compris, en fonction du nombre et de l'intensité de réalisation des opérations effectuées, entre 10 % et 99 %.

Les substances annexes précitées peuvent notamment comprendre des polysaccharides, en particulier des glucanes ou des mannanes, des protéines, des molécules d'ADN et d'ARN, des hydrolysats de polysaccharides de levure, en particulier de glycogène, de glucanes et de mannanes, des hydrolysats de protéines de levure et des saccharides tels que le tréhalose.

Le glycogène de levure ou l'extrait de levure riche en glycogène obtenu grâce à l'invention peut être utilisé soit sous sa forme brute (soluté aqueux) tel qu'il est obtenu par le procédé selon l'invention, soit sous des formes encapsulées ou immobilisées diverses telles que des nanovésicules (liposomes...), des nanoparticules (nanosphères), des microparticules (microsphères...) ou éventuellement sous des formes greffées par des lipides, protéines ou autres.

A titre d'exemples non limitatifs de réalisations pratiques de l'invention, on décrira ci-après quatre exemples de procédés de production et d'extraction de glycogène mettant en oeuvre les différentes opérations et traitements mentionnés précédemment.

### Exemple 1 :

Un procédé d'extraction de glycogène à partir de cellules de Saccharomyces carlsbergensis de fin de fermentation brassicole peut par exemple comprendre la suite d'opérations suivantes :
- Traitement thermique et chimique des cellules de levure pendant 1h30 dans NA₂CO₃ à 100° C ;
- Broyage des cellules par passage dans un homogénéisateur hautes pressions (à 900 bars) ;
- Centrifugation pendant 20 minutes à 4500 g ;
- Traitement du surnageant à l'éthanol (2,4 l/l) à 4° C pour précipiter le glycogène ou traitement du surnageant de broyat à l'isopropanol (2,4 l/l) à 4° C pour précipiter le glycogène ;
- Centrifugation et récupération du précipité.
Ce procédé permet d'obtenir une composition comprenant notamment 25 % de glycogène, 25 % de protéines et 20 % de cendres.

### Exemple 2 :

Un procédé de production de glycogène avec enrichissement initial des cellules de levure en glycogène et utilisant une levure de fin de fermentation brassicole peut par exemple comprendre la suite d'opérations suivantes :
- Lavage des cellules de levure à l'eau et centrifugation à 9500 t/mn ;
- Culture des cellules de levure dans un milieu contenant initialement 10 g/l de glucose, 0,5 g/l d'extrait de levure, 3 g/l de KH₂PO₄, 0,9 g/l de CaCl₂ et 1 g/l de MgSO₄. Dans un premier temps, la culture est réalisée de manière discontinue à 30° C avec un pH régulé à 4,5 par ajout d'ammoniaque 2N, et en aérobiose, avec un ensemencement de 50 g/l de cellules de levure. Dans un second temps, la culture est poursuivie par ajout d'une solution de 500 g/l de glucose contrôlé pour maintenir le niveau de glucose dans le fermenteur autour de 1 g/l (après 6 heures de culture, obtention de 55 g/l poids sec de levure à 20 % de glycogène);
- Récupération, lavage et centrifugation des cellules de levure ;
- Extraction du glycogène selon le procédé décrit dans l'Exemple 1.
Ce procédé permet d'obtenir une composition comprenant notamment 38 % de glycogène, 22 % de protéines et 23 % de cendres.

### Exemple 3 :

Un procédé de production de glycogène à partir d'une levure de brasserie propagé en fermenteur aérobie peut par exemple comprendre la suite d'opérations suivantes
- Production de levures Saccharomyces carlsbergensis par une fermentation aérobie. Milieu et conditions de culture identiques à celles de l'Exemple 2, à l'exception d'un ensemencement initial de cellules de levure à 1 g/l (après 48 heures de culture, obtention de 50 g/l de cellules de levure contenant 21 % de glycogène);
- Extraction de glycogène selon un procédé identique à celui décrit dans l'Exemple 1.

### Exemple 4 :

Un procédé de production de glycogène à partir de cellules de levure de fin de fermentation brassicole, comprenant un traitement de purification complémentaire par précipitation acide, peut par exemple être constitué par la suite d'opérations suivantes :
- Traitement thermique et chimique des cellules de levure pendant 1h30 dans Na₂CO₃ (0,25M) à 100° C ;
- Broyage des cellules par passage dans un homogénéisateur hautes pressions (1 passage à 900 bars) ;
- Centrifugation pendant 20 mn à 4500 g ;
- Traitement du surnageant à l'acide trichloroacétique (10 % p/V), centrifugation et élimination du précipité ;
- Traitement du surnageant à l'éthanol (2,4 l/l) à 4° C pour précipiter le glycogène ;
- centrifugation et récupération du précipité.
Ce procédé permet d'obtenir un composé comprenant notamment 64 % de glycogène et moins de 1 % de cendres.

La présente invention a également pour objet l'utilisation du glycogène ou d'un extrait riche en glycogène obtenu au moyen du procédé décrit ci-dessus, en tant que constituant d'un produit, d'une préparation ou d'une composition cosmétique, notamment destiné(e) à être appliqué(e) sur la peau.

Une telle composition cosmétique peut avantageusement comprendre du glycogène ou un extrait riche en glycogène obtenu tel que décrit précédemment, la teneur pondérale en glycogène de ladite composition étant comprise entre 0,001 % et 10 %.

De manière avantageuse, le glycogène ou l'extrait riche en glycogène utilisé est obtenu à partir de cellules de levure du type choisi dans le groupe formé par Saccharomyces carlsbergensis, Saccharomyces uvarum, Saccharomyces cerevisiae, Zygosaccharomyces fermentati, Candida utilis, Candida tropicalis, Hansunela anomala, Kluyveromyces fragilis, Debaromyces marana. Dekkera naardenensis, Geotrichum penicillatum, Lipomyces starkeyi, Metschnikowia lunata, Paschysolen tannophilus, Pichia abadieae et Torulopsis ernobii, préférentiellement des cellules de levure de fin de fermentation brassicole.

A titre d'exemples non limitatifs de réalisations pratiques de compositions selon l'invention, on décrira ci-après différent(e)s produits ou préparations cosmétiques comprenant du glycogène obtenu par l'intermédiaire du procédé conforme à l'invention.

### Exemple 1 :

Un produit cosmétique sous forme de crème pour les mains conforme à l'invention pourra, par exemple, présenter une composition pondérale, constituée à partir des fractions A, B et C suivantes, telle qu'indiquée ci-après.
Fraction A :
   - Alcool cétostéarylique (et) sulfate cétostéarylique de sodium 7,00 %
   - Acide stéarique 1,00 %
   - Octyldodécanol 3,00 %
Fraction B :
   - Eau distillée 48,40 %
   - Conservateur 0,30 %
   - Glycérine 40,00 %
   - Glycogène de cellules de levure 0,10 %
Fraction C :
   - Parfum 0,20 %
Le procédé de préparation et de fabrication de la crème pour les mains précitée consiste essentiellement à préparer la phase grasse A en la chauffant à 85° C, à chauffer ensemble l'eau et la glycérine à 75° C, à dissoudre le conservateur, puis à ajouter le glycogène sous agitation, à poursuivre celle-ci jusqu'à dispersion - dissolution parfaite, à verser A dans B sous agitation (au moyen d'une turbine), à refroidir progressivement le mélange A-B, à stopper, vers 50° C, la turbine et à poursuivre uniquement sous agitation planétaire et, enfin, à ajouter la fraction C (parfum) et à poursuivre l'agitation, jusqu'à refroidissement complet.

### Exemple 2 :

Un produit cosmétique sous forme de lotion tonique conforme à l'invention pourra, par exemple, présenter une composition pondérale, constituée par les fractions A, B et C, telle qu'indiquée ci-après.
Fraction A :
   - Alcool éthylique 3,000 %
   - Nonoxynol-14 0,600 %
   - Parfum 0,100%
Fraction B :
   - Propylène glycol 2,000 %
   - Glycérine 10,000 %
   - Conservateur 0,300 %
   - Eau distillée 73,735 %
   - Triéthanolamine 0,060 %
Fraction C :
   - Hamamélis Eau Distillée 9,500 %
   - Sodium Lactate 0,700 %
   - Glycogène de cellules de levure 0,005 %
Le procédé de préparation et de fabrication de la lotion tonique précitée consiste essentiellement à préparer la fraction B, par chauffage à + 45° C, sous agitation jusqu'à dissolution complète, à préparer séparément les solutés des fractions C et A par agitation à température ambiante, à mélanger ensemble les trois solutés A, B et C, et, enfin, à filtrer le mélange obtenu.

### Exemple 3 :

Un produit cosmétique sous forme de crème protectrice solaire conforme à l'invention pourra, par exemple, présenter une composition pondérale, constituée à partir des fractions A et B suivantes, telle qu'indiquée ci-après.
Fraction A :
   - Alcool cétylique 5,00 %
   - Triglycéride caprylique/caprique 9,00 %
   - Huile de paraffine 3,75 %
   - Huile de lanoline 1,00 %
   - Stéarate de glycérol 2,00 %
   - Diméthicone 0,25 %
   - Octyldodécanol 1,50 %
   - Benzophénone-3 4,50 %
   - Octyl methoxycinnamate 7,50 %
Fraction B :
   - Eau distillée 54,10 %
   - Conservateur 0,40 %
   - Glycérine 3,00 %
   - Phosphate cétylique de potassium 3,00 %
   - Glycogène de cellules de levure 5,00 %
Le procédé de préparation et de fabrication de la crème protectrice solaire précitée consiste essentiellement à préparer la phase grasse A par chauffage à 80° C, à préparer la phase aqueuse B par chauffage à + 75° C et agitation, à introduire la phase A dans la phase B sous agitation (au moyen d'une turbine), à poursuivre l'agitation et à effectuer un refroidissement jusqu'à 65° C, à stopper l'agitation turbine vers 50° C et à maintenir uniquement l'agitation planétaire et, enfin, à poursuivre l'agitation jusqu'au retour à température ambiante du mélange.

Bien entendu, l'invention n'est pas limitée au mode de réalisation décrit. Des modifications restent possibles, notamment du point de vue de la constitution des divers éléments ou par substitution d'équivalents techniques, sans sortir pour autant du domaine de protection de l'invention.

## Revendications

1. Procédé de production de glycogène ou d'un extrait riche en glycogène à partir de cellules de levure, **caractérisé en ce qu'**il consiste essentiellement à prendre une quantité donnée de cellules de levure, fournie par une culture spécifique ou récupérée en tant que résidus d'un procédé de fermentation, à soumettre lesdites cellules de levure à une opération d'enrichissement en glycogène intracellulaire en présence d'une source de carbone, à bloquer le métabolisme desdites cellules de levure, à désintégrer au moins partiellement les membranes desdites cellules de levure et à soumettre les substances intracellulaires libérées à une ou des précipitation(s) globale(s) ou sélectives). le cas échéant après application d'une opération de fractionnement, de concentration, de déminéralisation et/ou de décoloration.

2. Procédé selon la revendication 1, **caractérisé en ce que** l'opération d'enrichissement en glycogène comprend une première phase de revivification durant laquelle les cellules de levure sont disposées dans un milieu nutritif comprenant notamment une source de carbone, d'azote et de phosphore, des éléments minéraux et des vitamines, et une seconde phase de maturation durant laquelle les cellules de levure sont disposées dans un milieu nutritif contenant au moins une source de carbone, préférentiellement un sucre tel que le glucose ou le saccharose, à une concentration comprise entre 1 et 700 g par litre de milieu nutritif, une source d'azote, préférentiellement de l'ammoniaque et /ou divers éléments minéraux.

3. Procédé selon l'une quelconque des revendications 1 et 2, **caractérisé en ce que** le blocage du métabolisme des cellules de levure, en vue de la désactivation des processus enzymatiques de dégradation et de consommation endogène de glycogène, est obtenu en effectuant un traitement thermique à une température comprise entre 60° C et 180° C pendant une durée comprise entre 30 secondes et 200 minutes ou un traitement chimique au moyen du Na₂ CO₃ ou au moyen d'un solvant organique, tel que l'éthanol ou le propanol par exemple.

4. Procédé selon la revendication 3, **caractérisé en ce qu'**il consiste à effectuer, simultanément ou successivement, un traitement thermique et un traitement chimique des cellules de levure.

5. Procédé selon l'une quelconque des revendications 1 à 4, **caractérisé en ce que** la désintégration des parois des cellules de levure est réalisée par broyage mécanique par l'intermédiaire d'un homogénéisateur haute pression, d'un broyeur à billes ou d'une French presse.

6. Procédé selon l'une quelconque des revendications 1 à 4, **caractérisé en ce que** la désintégration des parois des cellules de levure est réalisée au moyen de traitements non mécaniques tels que des traitements thermiques constitués de cycles répétitifs de congélation/décongélation, des traitements enzymatiques mettant en oeuvre des préparations à base de protéases et d'hydrolases et des traitements chimiques au moyen de solvants organiques, mis en oeuvre seuls ou de manière combinée.

7. Procédé selon les revendications 5 et 6, **caractérisé en ce que** la désintégration des parois des cellules de levure est réalisée en combinant le broyage mécanique et au moins un type de traitement non mécanique.

8. Procédé selon l'une quelconque des revendications 1 à 7, **caractérisé en que** les substances intracellulaires libérées après désintégration des parois des cellules de levure sont soumises à au moins un traitement de fractionnement, de concentration et/ou de purification tel que la microfiltration, l'ultrafiltration ou la nanofiltration, l'électrodialyse, l'adsorption par échange d'ion ou l'adsorption par du charbon actif et la cryoconcentration.

9. Procédé selon l'une quelconque des revendications 1 à 8, **caractérisé en que** les substances intracellulaires libérées sont soumises à une précipitation par des acides, tels que l'acide chlorhydrique ou l'acide trichloroacétique, effectuée à un pH compris entre 1 et 6 et à une température comprise entre 0° C et 60° C.

10. Procédé selon l'une quelconque des revendications 1 à 8, **caractérisé en que** les substances intracellulaires libérées sont soumises à une précipitation par des sels, tels que le sulfate d'ammonium, effectuée à un pH compris entre 1 et 9, à une température comprise entre 0° C et 30° C et avec une teneur en sels comprise entre 10 % et 80 %.

11. Procédé selon l'une quelconque des revendications 1 à 8, **caractérisé en ce que** les substances intracellulaires libérées sont soumises à une précipitation par des alcools, préférentiellement l'éthanol ou l'isopropanol, effectuée à un pH compris entre 1 et 9, à une température comprise entre 0° C et 30° C et avec une teneur en alcool, en rapport volumique, comprise entre 10 % et 80 %.

12. Procédé selon l'une quelconque des revendications 1 à 11, **caractérisé en ce qu'**il consiste, avant l'opération d'enrichissement en glycogène, à soumettre les cellules de levure, notamment celles récupérées en tant que résidus d'un procédé de fermentation, à au moins un lavage à l'eau à une température comprise entre 10° C et 60° C, éventuellement avec addition d'un agent chimique tel que Na₂CO₃, puis à extraire et à concentrer lesdites cellules de levure, par centrifugation, décantation, filtration et/ou microfiltration.

13. Procédé selon l'une quelconque des revendications 1 à 12, **caractérisé en ce que** les substances intracellulaires libérées sont soumises à au moins un traitement d'hydrolyse enzymatique sélective effectué au moyen de préparations adaptées contenant des protéases, des glucanases, des mannases ou un mélange de deux ou de plusieurs des enzymes précitées.

14. Procédé selon l'une quelconque des revendications 1 à 13, **caractérisé en qu'**il consiste à concentrer, à stabiliser et à conditionner les compositions obtenues par précipitation.

15. Utilisation du glycogène ou de l'extrait riche en glycogène obtenu à partir de cellules de levure au moyen du procédé selon l'une quelconque des revendications 1 à 14, en tant que constituant d'une composition cosmétique, notamment destinée à être appliquée sur la peau.

16. Composition cosmétique comprenant du glycogène ou de l'extrait riche en glycogène obtenu à partir de cellules de levure au moyen du procédé selon l'une quelconque des revendications 1 à 14, la teneur pondérale en glycogène de ladite composition étant comprise entre 0,001 % et 10 %.

17. Composition cosmétique selon la revendication 16, **caractérisée en que** le glycogène ou l'extrait riche en glycogène est obtenu à partir de cellules de levure du type choisi dans le groupe formé par Saccharomyces carlsbergensis, Saccharomyces uvarum, Saccharomyces cerevisiae, Zygosaccharomyces fermentati, Candida utilis, Candida tropicalis, Hansunela anomala, Kluyveromyces fragilis, Debaromyces marana, Dekkera naardenensis, Geotrichum penicillatum, Lipomyces starkeyi, Metschnikowia lunata, Paschysolen tannophilus, Pichia abadieae et Torulopsis ernobii, préférentiellement des cellules de levure de fin de fermentation brassicole.

## Claims

1. Method for producing glycogen or an extract which is rich in glycogen from yeast cells, **characterised in that** it substantially consists of taking a given quantity of yeast cells provided by a specific culture or recovered as residues from a fermentation process, submitting said yeast cells to an intracellular glycogen enrichment operation in the presence of a carbon source, stopping the metabolism of said yeast cells, at least partially disintegrating the membranes of said yeast cells and submitting the liberated intracellular substances to one or more global or selective precipitations, if necessary after application of a fractionation, concentration, demineralisation and/or discolouration operation.

2. Method according to claim 1, **characterised in that** the glycogen enrichment operation comprises a first reactivation phase during which the yeast cells are disposed in a nutritive medium comprising in particular a source of carbon, nitrogen and phosphorus, mineral elements and vitamins, and a second maturation phase during which the yeast cells are disposed in a nutritive medium comprising at least one carbon source, preferably a sugar such as glucose or saccharose at a concentration between 1 and 700 g per litre of nutritive medium, a nitrogen source, preferably liquid ammonium and/or various mineral elements.

3. Method according to either one of claims 1 and 2, **characterised in that** the stopping of the yeast cell metabolism for deactivating the enzymatic processes of degradation and endogeneous consumption of glycogen is achieved by carrying out a thermal treatment at a temperature between 60°C and 180°C for a duration between 30 seconds and 200 minutes or a chemical treatment by means of Na₂CO₃ or by means of an organic solvent such as ethanol or propanol, for example.

4. Method according to claim 3, **characterised in that** it consists of carrying out, simultaneously or successively, a thermal treatment and a chemical treatment of the yeast cells.

5. Method according to any one of claims 1 to 4, **characterised in that** the disintegration of the yeast cell walls is achieved by mechanical grinding with the aid of a high pressure homogeniser, a ball grinder or a French press.

6. Method according to any one of claims 1 to 4, **characterised in that** the disintegration of the yeast cell walls is achieved by means of non-mechanical treatments such as thermal treatments consisting of repetitive freezing/defrosting cycles, enzymatic treatments using preparations based on proteinases and hydrolases and chemical treatments with organic solvents, used alone or in a combined manner.

7. Method according to claims 5 and 6, **characterised in that** the disintegration of the yeast cell walls is produced by combining the mechanical grinding and at least one type of non-mechanical treatment.

8. Method according to any one of claims 1 to 7, **characterised in that** after disintegration of the yeast cell walls, the liberated intracellular substances are subjected to at least one fractionation, concentration and/or purification treatment such as microfiltration, ultrafiltration or nanofiltration, electrodialysis, adsorption by ion exchange or adsorption by active carbon and cryoconcentration.

9. Method according to any one of claims 1 to 8, **characterised in that** the liberated intracellular substances are subjected to precipitation by acids, such as hydrochloric acid or trichloroacetic acid, carried out at a pH between 1 and 6 and at a temperature between 0°C and 60°C.

10. Method according to any one of claims 1 to 8, **characterised in that** the liberated intracellular substances are subjected to precipitation by salts, such as ammonium sulphate, and carried out at a pH between 1 and 9, at a temperature between 0°C and 30°C and with a salt content between 10% and 80%.

11. Method according to any one of claims 1 to 8, **characterised in that** the liberated intracellular substances are subjected to precipitation by alcohols, preferably ethanol or isopropanol, carried out at a pH between 1 and 9, at a temperature between 0°C and 30°C and with an alcohol content, by volume ratio, between 10% and 80%.

12. Method according to any one of claims 1 to 11, **characterised in that** it consists, prior to the glycogen enrichment operation, of subjecting the yeast cells, in particular cells which have been recovered as residues of a fermentation process to at least one washing in water at a temperature between 10°C and 60°C, optionally with the addition of a chemical agent such as Na₂CO₃, then extracting and concentrating said yeast cells, by centrifuging, decanting, filtration and/or microfiltration.

13. Method according to any one of claims 1 to 12, **characterised in that** the liberated intracellular substances are subjected to at least one selective enzymatic hydrolysis treatment carried out by means of suitable preparations containing proteinases, glucanases, mannases or a mixture of two or more of the above-mentioned enzymes.

14. Method according to any one of claims 1 to 13, **characterised in that** it consists of concentrating, stabilising and conditioning the compositions obtained by precipitation.

15. Use of glycogen or the extract which is rich in glycogen obtained from yeast cells by the method according to any one of claims 1 to 14, as constituting a cosmetic composition, in particular for application on the skin.

16. Cosmetic composition comprising glycogen or the extract which is rich in glycogen obtained from yeast cells by means of the method according to any one of claims 1 to 14, the percentage by weight of glycogen of said composition being between 0.001% and 10%.

17. Cosmetic composition according to claim 16, **characterised in that** the glycogen or the extract which is rich in glycogen is obtained from yeast cells of the type selected from the group formed by Saccharomyces carlsbergensis, Saccharomyces uvarum, Saccharomyces cerevisiae, Zygosaccharomyces fermentati, Candida utilis, Candida tropicalis, Hansunela anomala, Kluyveromyces fragilis, Debaromyces marana, Dekkera naardenensis, Geotrichum penicillatum, Lipomyces starkeyi, Metschnikowia lunata, Paschysolen tannophilus, Pichia abadieae and Torulopsis ernobii, preferably yeast cells from the end of brewing fementation.

## Patentansprüche

1. Verfahren zur Herstellung von Glykogen oder einem glykogenreichen Extrakt aus Hefezellen, **dadurch gekennzeichnet, daß** man im wesentlichen eine gegebene Menge Hefezellen nimmt, die von einer spezifischen Kultur geliefert oder als Fermentationsrückstand gewonnen wurde, diese Hefezellen in Gegenwart einer Kohlenstoffquelle mit intrazellulärem Glykogen anreichert, den Metabolismus dieser Hefezellen blockiert, die Membranen der Hefezellen zumindest teilweise zerstört und die freigesetzten intrazellulären Stoffe allgemein oder selektiv einoder mehrmals fällt, gegebenenfalls nach einem Fraktionierungs-, Konzentrations-, Entsalzungsund/oder Entfärbungsschritt.

2. Verfahren nach Anspruch 1, **dadurch gekennzeichnet, daß** dieser Arbeitsschritt der Glykogenanreicherung eine erste Belebungsphase, während der die Hefezellen in ein Nährmedium gegeben werden, das insbesondere eine Kohlenstoff-, Stickstoff- und Phosphorquelle, Mineralien und Vitamine enthält, und eine zweite Reifungsphase, während der die Hefezellen in ein Nährmedium gegeben werden, das mindestens eine Kohlenstoffquelle, vorzugsweise einen Zucker wie Glucose oder Saccharose, in einer Konzentration von 1 bis 700 g pro Liter Nährmedium, eine Stickstoffquelle, vorzugsweise wäßrigen Ammoniak, und/oder unterschiedliche Mineralien enthält, umfaßt.

3. Verfahren nach Anspruch 1 oder 2, **dadurch gekennzeichnet, daß** die Blockierung des Hefezellenmetabolismus zwecks Hemmung der enzymatischen Abbauprozesse und des endogenen Glykogenverbrauchs dadurch erzielt wird, daß man eine Hitzebehandlung von 30 Sekunden bis 200 Minuten bei 60°C bis 80°C oder eine chemische Behandlung mittels Na₂CO₃ oder mittels eines organischen Solvens wie zum Beispiel Ethanol oder Propanol durchführt.

4. Verfahren nach Anspruch 3, **dadurch gekennzeichnet, daß** man zugleich oder nacheinander eine Hitzebehandlung und eine chemische Behandlung der Hefezellen durchführt.

5. Verfahren nach einem der Ansprüche 1 bis 4, **dadurch gekennzeichnet, daß** man die Zerstörung der Hefezellwände durch mechanisches Zerkleinern mittels eines Hochdruckhomogenisators, einer Kugelmühle oder einer French-Presse durchführt.

6. Verfahren nach einem der Ansprüche 1 bis 4, **dadurch gekennzeichnet, daß** man die Zerstörung der Hefezellwände mittels nicht mechanischer Behandlungen wie Hitzebehandlungen, die aus wiederholten Frier-Tau-Zyklen bestehen, enzymatischer Behandlungen, bei denen Produkte auf Proteasen- und Hydrolasengrundlage verwendet werden, sowie chemischer Behandlungen mittels organischer Solventien durchführt, wobei diese Behandlungen getrennt oder in Kombination verwendet werden können.

7. Verfahren nach Anspruch 5 und 6, **dadurch gekennzeichnet, daß** man die Zerstörung der Hefezellwände durch Kombination der mechanischen Zerkleinerung mit mindestens einer Art der nicht mechanischen Behandlung durchführt.

8. Verfahren nach einem der Ansprüche 1 bis 7, **dadurch gekennzeichnet, daß** man die nach der Zerstörung der Hefezellwände freigesetzten intrazellulären Stoffe mindestens einer Fraktionierungs-, Konzentrations- und/oder Reinigungsbehandlung wie Mikrofiltration, Ultrafiltration oder Nanofiltration, Elektrodialyse, Ionenaustauschadsorption, Aktivkohleadsorption und Kryokonzentration unterzieht.

9. Verfahren nach einem der Ansprüche 1 bis 8, **dadurch gekennzeichnet, daß** die freigesetzten intrazellulären Stoffe einer Säurefällung, wie z.B. einer Fällung mit Salzsäure oder Trichloressigsäure, und zwar bei einem pH von 1 bis 6 und einer Temperatur von 0°C bis 60°C unterzogen werden.

10. Verfahren nach einem der Ansprüche 1 bis 8, **dadurch gekennzeichnet, daß** die freigesetzten intrazellulären Stoffe einer Salzfällung, wie z.B. mit Ammoniumsulfat, bei einem pH von 1 bis 9, einer Temperatur von 0°C bis 30°C und mit einem Salzgehalt von 10% bis 80% unterzogen werden.

11. Verfahren nach einem der Ansprüche 1 bis 8, **dadurch gekennzeichnet, daß** die freigesetzten intrazellulären Stoffe einer Alkoholfällung, vorzugsweise mit Ethanol oder Isopropanol, bei einem pH von 1 bis 9, einer Temperatur von 0°C bis 30°C mit einem Alkoholgehalt von 10 Vol.-% bis 80 Vol.-% unterzogen werden.

12. Verfahren nach einem der Ansprüche 1 bis 11, **dadurch gekennzeichnet, daß** es vor dem Arbeitsschritt der Glykogenanreicherung daraus besteht, daß man die Hefezellen, insbesondere die als Fermentationsrückstand gewonnenen Hefezellen, mindestens einer Waschung mit Wasser bei einer Temperatur von 10°C bis 60°C, gegebenenfalls unter Zusatz einer Chemikalie wie Na₂CO₃, unterzieht, dann diese Hefezellen extrahiert und mittels Zentrifugation, Abdekantieren, Filtration und/oder Mikrofiltration konzentriert.

13. Verfahren nach einem der Ansprüche 1 bis 12, **dadurch gekennzeichnet, daß** man die freigesetzten intrazellulären Stoffe mindestens einer selektiven enzymatischen Aufschlußbehandlung mittels geeigneter Präparate, die Proteasen, Glucanasen, Mannasen oder eine Mischung aus zwei oder mehreren der genannten Enzyme enthalten, unterzieht.

14. Verfahren nach einem der Ansprüche 1 bis 13, **dadurch gekennzeichnet, daß** man die durch Fällung erhaltenen Zusammensetzungen konzentriert, stabilisiert und verpackt.

15. Verwendung des aus Hefezellen gewonnenen und nach einem Verfahren nach Anspruch 1 bis 14 erhaltenen Glykogens oder glykogenreichen Extrakts als Bestandteil einer Kosmetikzusammensetzung, insbesondere zum Auftragen auf die Haut.

16. Kosmetikzusammensetzung, enthaltend aus Hefezellen gewonnenes und mittels des Verfahrens nach einem der Ansprüche 1 bis 14 erhaltenes Glykogen oder glykogenreichen Extrakt, wobei der Glykogengehalt dieser Zusammensetzung zwischen 0,001 Gew.-% and 10 Gew.-% liegt.

17. Kosmetikzusammensetzung nach Anspruch 16, **dadurch gekennzeichnet, daß** das Glykogen oder der glykogenreiche Extrakt aus Hefezellen der aus der Gruppe Saccharomyces carlsbergensis, Saccharomyces uvarum, Saccharomyces cerevisiae, Zygosaccharomyces fermentati, Candida utilis, Candida tropicalis, Hansunela anomala, Kluyveromyces fragilis, Debaromyces marana, Dekkera naardenensis, Geotrichum penicillatum, Lipomyces starkeyi, Metschnikowia lunata, Paschysolen tannophilus, Pichia abadieae und Torulopsis ernobii gewählten Art gewonnen wird, vorzugsweise aus Hefezellen aus Brauereirückständen.
